# EUROPÄISCHE PATENTANMELDUNG

(11) **EP 4 631 543 A1**
(43) Veröffentlichungstag der Anmeldung: **15.10.2025**
(21) Anmeldenummer: 24169701.0
(22) Anmeldetag: 11.04.2024
(51) Int. Cl.: A61M 1/06

(54) **BRUSTPUMPE**

(71) Anmelder: MAM Baby AG, 8832 Wollerau (CH)
(72) Erfinder: Die Erfindernennung liegt noch nicht vor
(74) Vertreter: SONN Patentanwälte GmbH & Co KG

(57) **Zusammenfassung**

Brustpumpe (1), aufweisend
- einen Milchbehälter (2) mit einem Hohlraum (3) zur Aufnahme von abgepumpter Milch;
- eine Brusthaube (4), die einen Brusttrichter (5) und einen Nippeltunnel (6) aufweist;
- ein Verbindungselement (7), das eine Öffnung (8) zum Hohlraum (3) des Milchbehälters (2) mit dem Nippeltunnel (6) verbindet;
- einen Entlüftungskanal (9), dessen erstes Ende (10) zum Hohlraum (3) des Milchbehälters (2) offen ist und dessen zweites Ende (11) offen zur Umgebung ist, wobei zumindest ein Abschnitt des Entlüftungskanals (9) entlang des Verbindungselements (7) verläuft.

## Beschreibung

Die Erfindung betrifft eine Brustpumpe, aufweisend einen Milchbehälter mit einem Hohlraum zur Aufnahme von abgepumpter Milch; eine Brusthaube, die einen Brusttrichter und einen Nippeltunnel aufweist; ein Verbindungselement, das eine Öffnung zum Hohlraum des Milchbehälters mit dem Nippeltunnel verbindet.

Eine solche Brustpumpe ist beispielsweise in der EP 4066870 A2 gezeigt.

Der Brusttrichter wird auf die Brust aufgesetzt. Durch die Erzeugung eines Unterdrucks wird Milch aus der Brust abgepumpt, die über den Verbindungselement in den Hohlraum des Milchbehälters fließt. Wenn die Milch in den Milchbehälter gepumpt wird, würde in diesem ein Überdruck entstehen, der das weitere Abpumpen behindert (vgl. Fig. 13 der EP 4066870 A2). Dies ist insbesondere ein Problem bei Brustpumpen, bei denen das Pumpelement keine Luft in die Umgebung abgibt, sondern zyklisch arbeitet bzw. zyklisch Luft ansaugt, beispielsweise eine zyklische Membranpumpe (die den Vorteil hat, dass sie unempfindlich gegen Verunreinigungen durch die Milch und gegen Dauerbeanspruchung ist) und wenn der Milchbehälter starr ausgeführt ist. Daher ist es bekannt, dass der Milchbehälter oder ein Anschlusselement des Verbindungselements mit dem Milchbehälter eine oder mehrere Bohrungen aufweist, durch die Luft nach außen dringen kann und die der Entlüftung des Milchbehälters dient. Solche Anordnungen werden beispielsweise bei Brustpumpen verwendet, die (unter der Kleidung) tragbar sind.

Nachteiligerweise kann durch die Entlüftungsbohrung Milch aus dem Milchbehälter austreten. Daher wird die Bohrung üblicherweise möglichst weit oben (in der Betriebsstellung der Brustpumpe beim Abpumpen) vorgesehen, d.h. insbesondere am Anschlusselement des Verbindungselements mit dem Milchbehälter. Wenn die Brustpumpe schief gehalten wird oder sich die abpumpende Person vorbeugt, kann jedoch dennoch Milch austreten und verloren gehen.

Es ist eine Aufgabe der vorliegenden Erfindung, einen oder mehrere der Nachteile des Standes der Technik zu lindern oder zu beseitigen. Insbesondere ist es eine Aufgabe der vorliegenden Erfindung, einen Austritt von Milch aus der Brustpumpe, insbesondere bei einer Schrägstellung der Brustpumpe, zu verringern.

Dies wird gelöst durch eine Brustpumpe wie eingangs angeführt, wobei die Brustpumpe einen Entlüftungskanal aufweist, dessen erstes Ende zum Hohlraum des Milchbehälters offen ist und dessen zweites Ende offen zur Umgebung (Atmosphäre) ist, wobei zumindest ein Abschnitt des Entlüftungskanal entlang des Verbindungselements verläuft.

Durch den Entlüftungskanal kann der Überdruck im Hohlraum abgebaut werden. Da der Entlüftungskanal entlang des Verbindungselements führt und das zweite offene Ende vom ersten offenen Ende beabstandet ist, tritt weniger Milch (als bei einer Bohrung vom Hohlraum zur Umgebung) aus, da zuerst der Weg im Entlüftungskanal zurückgelegt werden muss, insbesondere wenn dieser Weg teilweise nach oben führt. Außerdem muss die Milch bei Schräghaltung der Brustpumpe weiter steigen, bis sie das zweite offene Ende erreicht. Selbst wenn das zweite Ende unterhalb des ersten Endes liegen sollte, wird der Ausfluss an Milch reduziert, da der Durchfluss durch den Kanal begrenzt ist.

Das zweite Ende stellt vorzugsweise eine Entlüftungsöffnung dar. Bevorzugt liegt das zweite Ende in einer Betriebsstellung der Brustpumpe zum Abpumpen von Milch oberhalb des ersten Endes. Das zweite Ende ist bevorzugt zumindest 2 mm, besonders bevorzugt zumindest 5 mm, noch mehr bevorzugt zumindest 1 cm oder 1,5 cm, vom ersten Ende beabstandet, und/oder liegt vorzugsweise zumindest entsprechend diesen Werten oberhalb des ersten Endes. Der Entlüftungskanal hat eine Länge von bevorzugt mehr als 2 mm, besonders bevorzugt mehr als 5 mmm, noch mehr bevorzugt mehr als 1 cm. Der Entlüftungskanal weist ein Volumen von bevorzugt mehr als 5 mm^3, besonders bevorzugt mehr als 10 mm^3, noch mehr bevorzugt mehr als 20 mm^3, auf. Durch ein höheres Volumen kann der Milchaustritt weiter reduziert werden. Das zweite Ende des Entlüftungskanals weist einen Öffnungsquerschnitt mit einer Fläche von bevorzugt weniger als 25 mm^2, besonders bevorzugt weniger als 10 mm^2, noch mehr bevorzugt weniger als 5 mm^2 oder 2 mm^2 auf. Durch einen geringeren Austrittsquerschnitt kann der Milchaustritt weiter reduziert werden. Der Entlüftungskanal ist bis auf das erste und das zweite Ende bevorzugt flüssigkeitsdicht. Vorzugsweise erstreckt sich der Entlüftungskanal zumindest abschnittsweise entlang einer Wand des Verbindungselements. Der Entlüftungskanal stellt somit insbesondere nicht einfach nur eine Bohrung senkrecht durch eine Wand dar, sondern erstreckt sich insbesondere zumindest abschnittsweise in eine Wanderstreckungsrichtung bzw. entlang einer Flächenrichtung der Wand (wobei die Flächenrichtung normal zur Dickenerstreckung der Wand ist). Das erste Ende des Entlüftungskanals ist von der Öffnung zwischen Verbindungselement und Milchbehälter verschieden. Insbesondere ist das zweite Ende des Entlüftungskanals in der Betriebsstellung der Brustpumpe zum Abpumpen zur Umgebung offen (und insbesondere nicht von der Brust verdeckt).

Das Verbindungselement weist vorzugsweise einen Verbindungsabschnitt auf, der zur Verbindung mit dem Nippeltunnel vorgesehen ist bzw. der den Nippeltunnel aufnimmt. Der Verbindungsabschnitt ist insbesondere allgemein zylinderförmig oder konisch ausgebildet. Die Brusthaube ist vorzugsweise mit dem Nippeltunnel in das Verbindungselement einsteckbar und wird in diesem durch Reibschluss gehalten. Vorzugsweise weist der Nippeltunnel im aus dem Verbindungsabschnitt entfernten Zustand einen Außenumfang auf, der gleich oder größer als ein Innenumfang des Verbindungsabschnitts ist. Durch die Übermäßigkeit des Nippeltunnels ist dieser vorteilhafterweise zum Verbindungselement dichtend. Vorzugsweise weist der Nippeltunnel ein weicheres Material (insbesondere ein Material mit einer niedrigeren Shore-A-Härte) auf als der allgemein zylindrische Abschnitt. Vorzugsweise ist die Brusthaube vom Verbindungselement zerstörungsfrei trennbar. Der Verbindungsabschnitt hat vorzugsweise die Form eines allgemeinen Zylinders, insbesondere eines Kreiszylinders oder eines elliptischen Zylinders. Der Nippeltunnel hat vorzugsweise die Form eines allgemeinen Zylinders oder eines Kegelstumpfes (Konus). Der Verbindungsabschnitt des Verbindungselements ist vorzugsweise an den Nippeltunnel angepasst. Statt der allgemein zylindrischen Form kann auch eine kegelstumpfförmige oder pyramidenstumpfförmige Form für den Nippeltunnel und/oder den Verbindungsabschnitt vorgesehen sein. Weiters weist das Verbindungselement vorzugsweise ein Anschlusselement zur Verbindung mit dem Milchbehälter auf. Das Anschlusselement ist bevorzugt allgemein zylindrisch.

Der Milchbehälter ist vorzugsweise mit dem Verbindungselement verbindbar, z.B. am Verbindungselement anschraubbar, ansteckbar oder über einen Bajonettverschluss verbindbar. Vorzugsweise weist das Anschlusselement eine axiale Erstreckungsrichtung auf, die in einem Winkel zwischen 45° und 135°, weiter bevorzugt zwischen 60° und 120°, besonders bevorzugt im Wesentlichen senkrecht, zu einer axialen Erstreckungsrichtung des Verbindungsabschnitts steht. Bevorzugt verläuft der Entlüftungskanal zumindest abschnittsweise entlang des Verbindungselements in eine Richtung weg vom Anschlusselement und/oder in eine Richtung, die einen Winkel von weniger als 30° zu einer axialen Erstreckungsrichtung des Anschlusselements aufweist. Darunter, dass der Verbindungsabschnitt bzw. der Nippeltunnel allgemein zylinderförmig oder konisch ausgebildet sind, wird insbesondere verstanden, dass diese die Form der Mantelfläche eines allgemeinen Zylinders bzw. Konus (d.h. Kegelstumpfes) aufweisen.

Der Brusttrichter ist insbesondere zur (teilweisen) Aufnahme einer Brust eingerichtet. Der Milchbehälter ist vorzugsweise starr ausgebildet. Vorzugweise verläuft ein Milchflussweg vom Nippeltunnel bzw. von der Brusthaube über das Verbindungselement zum Hohlraum des Milchbehälters, wobei der Milchflussweg insbesondere durch ein Ventil verläuft, das vorzugsweise den Vakuumaufbau im Nippeltunnel ermöglicht, bspw. das unten angeführte Rückschlagventil. Beim Abpumpen wird die abgepumpte Milch entlang des Milchflusswegs geführt. Vorzugsweise ist eine Pumpe zur Erzeugung eines Unterdrucks in der Brusthaube vorgesehen. Vorzugsweise bildet das Verbindungselement im Wesentlichen einen Milchflusskanal, der den Nippeltunnel mit dem Hohlraum verbindet. Vorzugsweise weist der Milchflusskanal eine Öffnung auf, über die ein Pumpenelement einen Unterdruck im Verbindungselement bzw. im Milchflusskanal erzeugen kann. Vorzugsweise ist eine Membran vorgesehen, über die das Pumpenelement über die Öffnung einen Unterdruck im Verbindungselement bzw. im Milchflusskanal erzeugen kann.

Bevorzugt weist der Nippeltunnel auf seinem äußeren Umfang eine Dichtgeometrie auf, bevorzugt zumindest eine oder zumindest zwei (insbesondere in Umfangsrichtung verlaufende) Dichtrippe(n) auf. Als Dichtgeometrie können bspw. (z.B. zusätzlich oder alternativ zu der/den Dichtrippen) insbesondere umlaufende Lamellen vorgesehen sein. Vorzugsweise ist der Entlüftungskanal zumindest abschnittsweise durch zwei Dichtrippen begrenzt. D.h. insbesondere, dass der Bereich zwischen den zwei Dichtrippen einen Abschnitt des Entlüftungskanal bildet. Die zwei Dichtrippen sind dabei vorzugsweise vollumfänglich ausgebildet.

Die Brustpumpe ist vorzugsweise eine elektrische Brustpumpe.

Der Milchbehälter ist vorzugsweise starr ausgeführt, sodass das Volumen des Hohlraums beim Abpumpen im Wesentlichen unverändert bleibt. Somit ist der Milchbehälter vorzugsweise kein flexibler Beutel.

Vorzugsweise ist das zweite Ende des Entlüftungskanals weiter vom Milchbehälter entfernt als das erste Ende. Vorzugsweise ist das zweite Ende beabstandet vom Milchbehälter. Dadurch wird die Distanz gesteigert, die Milch den Entlüftungskanal füllen muss, bevor diese austritt. Vorzugsweise liegt das zweite Ende nicht auf einem Wandabschnitt des Milchbehälters.

Es ist vorteilhaft, wenn zumindest ein Abschnitt des Entlüftungskanals in eine Richtung weg vom Hohlraum verläuft.

Vorzugsweise verläuft der Entlüftungskanal zumindest abschnittsweise in Mäanderform entlang des Verbindungselements. Dadurch entsteht ein längerer Fließweg mit größerem Volumen, wodurch die Zeit bis zu einem etwaigen Austritt von Milch durch das zweite Ende vergrößert wird.

Es ist bevorzugt, wenn der Entlüftungskanal zumindest abschnittsweise entlang eines Abschnitts verläuft, an dem das Verbindungselement den Nippeltunnel aufnimmt und/oder mit dem Nippeltunnel in Kontakt steht. Somit liegt das zweite Ende in der Betriebsstellung höher bzw. ist vom Hohlraum weiter entfernt.

Es ist bevorzugt, wenn das erste Ende des Entlüftungskanals durch eine Öffnung (insbesondere eine Bohrung) in einem Wandabschnitt des Verbindungselements gebildet ist, wobei die Öffnung auf der einen Seite zum Hohlraum und auf der anderen Seite zum Nippeltunnel, insbesondere zu einem äußeren Umfang des Nippeltunnels, mündet. Damit kann auf einfache Weise der Entlüftungskanal in einem Raum zwischen Nippeltunnel und Verbindungselement vom Hohlraum weggeführt werden. Vorzugsweise ist die Öffnung/Bohrung im Verbindungsabschnitt des Verbindungselements vorgesehen. Vorzugsweise mündet die Öffnung/Bohrung insbesondere zu dem Bereich zwischen den beiden Dichtrippen des Nippeltunnels, die einen Abschnitt des Entlüftungskanals bilden können.

Es ist vorteilhaft, wenn das Verbindungselement einen Verbindungsabschnitt zur Verbindung mit dem (bzw. zur Aufnahme und/oder zum Einstecken des) Nippeltunnel(s) aufweist, wobei der Verbindungsabschnitt allgemein zylinderförmig oder konisch ausgebildet ist, wobei der Entlüftungskanal zumindest abschnittsweise in Umfangsrichtung des Verbindungsabschnitts des Verbindungselements verläuft. Vorzugsweise verläuft der Entlüftungskanal in Umfangsrichtung über einen Winkelbereich von zumindest 45°, bevorzugt zumindest 90°, noch mehr bevorzugt zumindest 120° oder 150°, des Umfangs des (insbesondere kreiszylinderförmigen) Verbindungsabschnitts.

Vorzugsweise ist der Entlüftungskanal zumindest abschnittsweise durch eine Nut im Nippeltunnel gebildet (insbesondere in einer äußeren Mantelfläche des Nippeltunnels) und/oder durch eine Nut in einer inneren Mantelfläche des Verbindungselements. Vorzugsweise verläuft die Nut zumindest abschnittsweise mäanderförmig.

Es ist bevorzugt, wenn der Entlüftungskanal zumindest abschnittsweise gebildet ist durch eine (insbesondere einen Abschnitt der bereits oben erwähnten) Nut im Nippeltunnel (insbesondere einer äußeren Mantelfläche des Nippeltunnels), die in Umfangsrichtung des Nippeltunnels verläuft, wobei vorzugsweise die andere Seite der Öffnung (die das erste Ende bildet) zur Nut mündet. Damit kann auf einfache Weise der Entlüftungskanal bereitgestellt und vom Hohlraum weggeführt werden. Es kann auch (alternativ oder zusätzlich) eine (in Umfangsrichtung verlaufende) Nut im Innenumfang (d.h. in der inneren Mantelfläche) des Verbindungselements vorgesehen sein. Vorzugsweise erstreckt sich die Nut vollumfänglich. Dadurch kann die Produktion vereinfacht werden.

Es ist bevorzugt, wenn der Entlüftungskanal zumindest abschnittsweise in axialer Richtung des Verbindungsabschnitts des Verbindungselements verläuft. Dadurch verläuft der Entlüftungskanal in der Betriebsstellung beim Abpumpen näher an der benützenden Person heran. Wenn sich die benützende Person nach vorne beugt, verläuft dieser (axial verlaufende) Abschnitt somit nach oben, womit ein Milchaustritt verhindert wird. Bevorzugt verläuft der Entlüftungskanal also abschnittsweise in Richtung der benützenden Person. Vorzugsweise schließt der in axiale Richtung verlaufende Abschnitt des Entlüftungskanals an den in Umfangsrichtung verlaufenden Abschnitt des Entlüftungskanals an (oder umgekehrt).

Es ist vorteilhaft, wenn der Entlüftungskanal zumindest abschnittsweise gebildet ist durch eine (insbesondere einen Abschnitt der bereits oben erwähnten) Nut im Nippeltunnel (insbesondere an einer äußeren Mantelfläche des Nippeltunnels), die in axialer Richtung des Verbindungsabschnitts des Verbindungselements verläuft. Falls der Nippeltunnel umfänglich verlaufende Dichtrippen aufweist, weisen diese insbesondere ggf. Unterbrechungen für die Nut auf. Es kann auch (alternativ oder zusätzlich) eine (axial verlaufende) Nut im Innenumfang des Verbindungselements vorgesehen sein. Die axial verlaufende Nut ist insbesondere auf der der Öffnung zum Hohlraum im Wandabschnitt des Verbindungselements gegenüberliegenden Seite des Verbindungsabschnitts vorgesehen. Insbesondere wenn ein Abschnitt des Entlüftungskanals durch einen Bereich zwischen den zwei Dichtrippen des Nippeltunnels gebildet ist, weist vorzugsweise eine der Dichtrippen eine Nut auf, um einen axial verlaufenden Abschnitt des Entlüftungskanals zu bildet.

Vorzugsweise ist das zweite Ende des Entlüftungskanals gebildet durch (zumindest) eine Bohrung im Verbindungsabschnitt des Verbindungselements, wobei die Bohrung vorzugsweise auf der einen Seite zur Nut im Nippeltunnel (und/oder Verbindungselement) und/oder zum Bereich zwischen den Dichtrippen und auf der anderen Seite zur Umgebung mündet. Die Bohrung kann radial und/oder tangential verlaufen. Dies ermöglicht eine einfache Entformung durch seitliche Schieber im Werkzeug.

Insbesondere alternativ zur Bohrung ist es vorteilhaft, wenn der Entlüftungskanal zu einer umfänglichen Kante des Verbindungsabschnitts des Verbindungselements verläuft, die mit dem Nippeltunnel in Kontakt steht, wobei die Kante des Verbindungsabschnitts und/oder der Nippeltunnel eine radial verlaufende Nut aufweist, über die der Entlüftungskanal zur Umgebung mündet. Das zweite Ende des Entlüftungskanals wird somit durch die radial verlaufende Nut gebildet.

Vorzugsweise verläuft der Entlüftungskanal zumindest abschnittsweise entlang des Verbindungsabschnitts des Verbindungselements, wobei der Verlauf des Entlüftungskanals eine axiale Komponente und eine in Umfangsrichtung verlaufende Komponente aufweist (also bspw. schräg oder entlang einer Helix verläuft). Vorzugsweise ist der Entlüftungskanal zumindest abschnittsweise durch eine Nut des Nippeltunnels gebildet, wobei der Verlauf der Nut eine axiale Komponente und eine in Umfangsrichtung verlaufende Komponente aufweist (also bspw. schräg oder entlang einer Helix oder mäanderförmig verläuft). Der Entlüftungskanal bzw. die Nut (im Nippeltunnel und/oder Verbindungselement) kann beispielsweise zumindest abschnittsweise gebildet sein durch eine (an das erste Ende anschließende) in Umfangsrichtung verlaufende und anschließend axial verlaufende Komponente oder durch eine (an das erste Ende anschließende) axial verlaufende, anschließende in Umfangsrichtung verlaufende und anschließend axial verlaufende Komponente; daran kann jeweils eine radial verlaufende Komponente anschließen.

Es ist auch möglich, dass der Entlüftungskanal zumindest abschnittsweise gebildet ist durch einen Bereich zwischen dem Verbindungselement und dem Nippeltunnel (insbesondere zwischen einer inneren Mantelfläche des Verbindungselements und einer äußeren Mantelfläche des Nippeltunnels), wobei der Bereich gebildet ist durch unterschiedliche Konuswinkel des Verbindungselements und des Nippeltunnels (insbesondere der inneren Mantelfläche des Verbindungselements und der äußeren Mantelfläche des Nippeltunnels). Dabei weist vorzugsweise die äußere Mantelfläche des Nippeltunnels einen kleineren Konuswinkel als die innere Mantelfläche des Verbindungselements auf. Unter dem Konuswinkel wird dabei der Winkel zwischen der jeweiligen Mantellinie und der jeweiligen Kegelachse verstanden.

Der Entlüftungskanal bzw. zumindest ein Abschnitt des Entlüftungskanals kann aus mehreren der in dieser Offenbarung erwähnten Abschnitte bzw. Ausführungsformen gebildet sein.

Es ist bevorzugt, wenn das zweite Ende des Entlüftungskanals in einer (aufrechten) Betriebsstellung der Brustpumpe zum Abpumpen von Milch oberhalb des ersten Endes des Entlüftungskanals liegt. In der Betriebsstellung ist der Milchbehälter typischerweise unterhalb des Nippeltunnels vorgesehen. Insbesondere hat die Öffnung des Behälters eine axiale Achse, die im Wesentlichen senkrecht zur axialen Achse des Nippeltunnels steht und senkrecht zur Horizontalen ist.

Vorteilhafterweise ist ein Rückschlagventil (Einwegventil) an der Öffnung zum Hohlraum des Milchbehälters vorgesehen. Vorzugsweise weist das Verbindungselement das Rückschlagventil auf. Vorzugsweise weist das Verbindungselement eine Dichtfläche auf, die den Hohlraum bis auf das Rückschlagventil abdichtet. Vorzugsweise weist die Dichtfläche das erste Ende des Entlüftungskanals auf. Das Rückschlagventil ist vorzugsweise als Schnabelventil ausgebildet. Das Rückschlagventil verhindert, dass Milch aus dem Hohlraum in Richtung des Verbindungselements austreten oder zurückfließen kann. Vorzugsweise liegt das erste Ende näher an der Brusthaube als das Rückschlagventil, insbesondere ist das erste Ende zwischen Brusthaube und Rückschlagventil vorgesehen. Dadurch liegt das erste Ende bei einem Vorbeugen der benützenden Person weiter oben.

Es ist bevorzugt, wenn das Verbindungselement eine Membran aufweist, über die ein Pumpelement einen Unterdruck im Verbindungselement erzeugen kann.

Es ist bevorzugt, wenn die Brustpumpe ein elektrisches Pumpenelement aufweist, das über die Membran einen Unterdruck im Verbindungselement erzeugen kann. Vorzugsweise weist die Brustpumpe einen Energiespeicher auf, der mit dem elektrischen Pumpelement verbunden ist.

Die Erfindung wird im Folgenden anhand eines in den Figuren dargestellten bevorzugten Ausführungsbeispiels näher erläutert, das für die Erfindung jedoch nicht einschränkend ist.
Fig. 1 zeigt schematisch eine bevorzugte Ausführungsform einer erfindungsgemäßen Brustpumpe (ohne einem Pumpelement) in einem Querschnitt.
Fig. 2 zeigt schematisch einen Detailausschnitt eines Verbindungselements der Brustpumpe in der Ausführungsform der Fig. 1 in einer Schrägansicht.
Fig. 3 zeigt schematisch einen Detailausschnitt einer Brusthaube der Brustpumpe in der Ausführungsform der Fig. 1 in einer Schrägansicht.
Fig. 4 zeigt schematisch einen Detailausschnitt der Brusthaube der Brustpumpe in der Ausführungsform der Fig. 1 in einem Querschnitt.

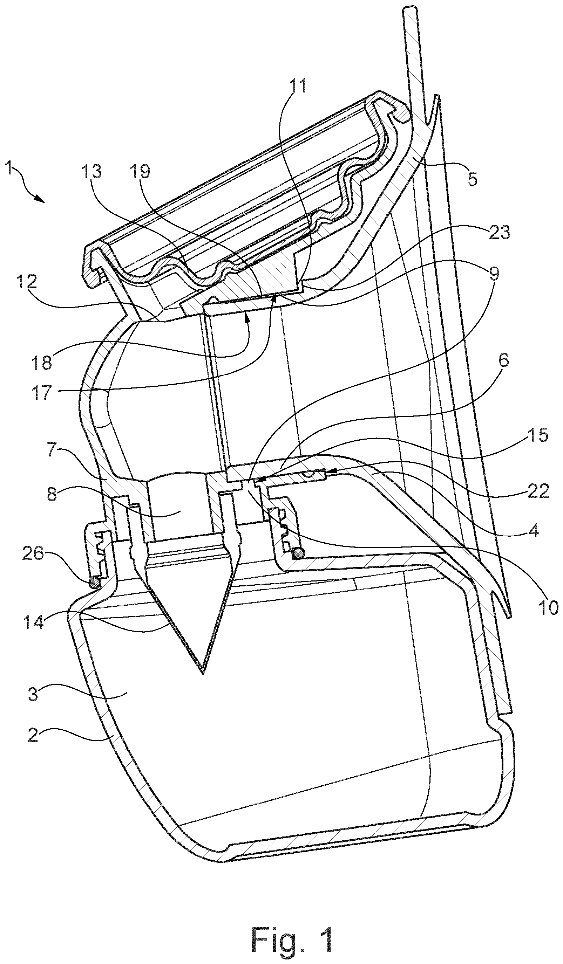

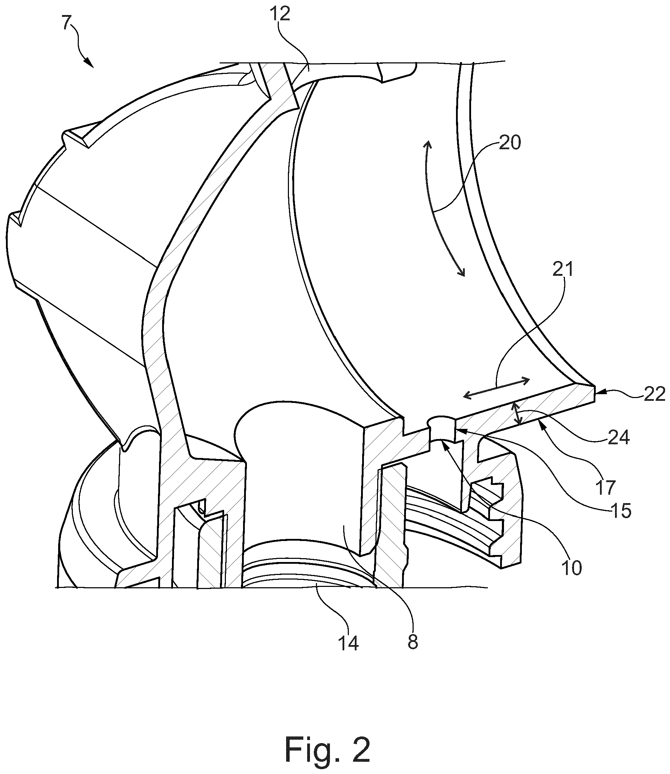

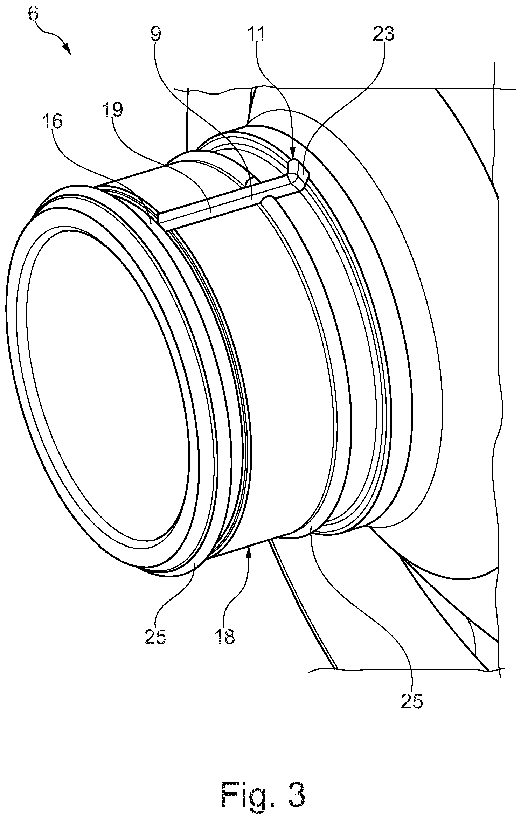

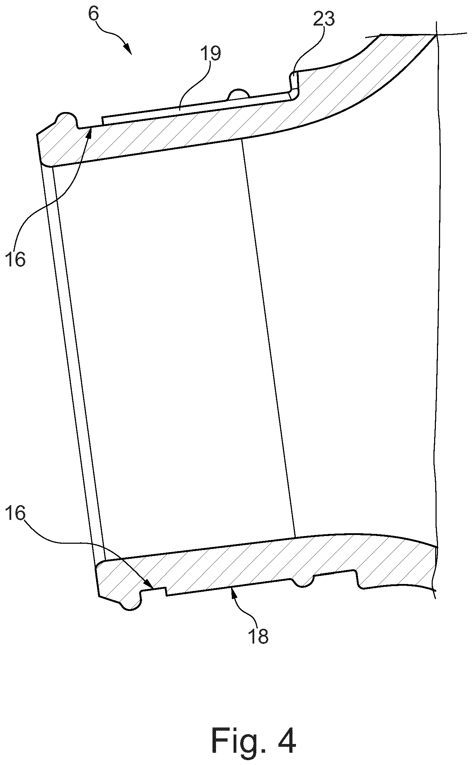

Fig. 1 zeigt schematisch eine bevorzugte Ausführungsform einer Brustpumpe 1 (ohne einem Pumpelement) in einem Querschnitt. Die Brustpumpe 1 weist einen Milchbehälter 2 mit einem Hohlraum 3 zur Aufnahme von abgepumpter Milch, eine Brusthaube 4, die einen Brusttrichter 5 und einen Nippeltunnel 6 aufweist, und ein Verbindungselement 7, das eine Öffnung 8 zum Hohlraum 3 des Milchbehälters 2 mit dem Nippeltunnel 6 verbindet, auf. Das Verbindungselement 7 bildet somit einen Kanal, der den Nippeltunnel 6 und den Hohlraum 3 verbindet und durch den die abgepumpte Milch in den Hohlraum 3 gelangt, um dort gelagert zu werden. Das Verbindungselement 7 weist einen Verbindungsabschnitt 17 zur Verbindung mit dem Nippeltunnel 6 auf, wobei der Verbindungsabschnitt 17 insbesondere allgemein zylinderförmig oder konisch ausgebildet ist, in dem der Nippeltunnel 6 insbesondere eingesteckt ist, wobei der Nippeltunnel ebenfalls einen Verbindungsabschnitt 18 (der insbesondere allgemein zylinderförmig oder konisch ausgebildet ist) aufweist. In dieser Ausführungsform sind die Verbindungsabschnitte 17, 18 jeweils im Wesentlichen kreiszylinderförmig bzw. konisch. Fig. 2 zeigt schematisch einen Detailausschnitt des Verbindungselements 7 in einer Schrägansicht. Fig. 3 zeigt schematisch einen Detailausschnitt der Brusthaube 4 in einer Schrägansicht. Fig. 4 zeigt schematisch einen Detailausschnitt der Brusthaube 4 in einem Querschnitt.

Das Verbindungselement, insbesondere dieser Kanal, weist einen Durchlass 12 zu einem weiteren Raum auf, der von einer Membran 13 überspannt ist. An dieser Membran greift ein (insbesondere elektrisches) Pumpelement (nicht gezeigt) der Brustpumpe 1 an, um zyklisch einen Unterdruck im Verbindungselement 7 zu erzeugen, und damit Milch aus der Brust abzupumpen. In der Betriebsstellung zum Abpumpen wird die Brust einer Person vom Brusttrichter 5 und deren Nippel vom Nippeltunnel 6 aufgenommen.

Die Brustpumpe 1 weist außerdem ein Rückschlagventil 14 an der Öffnung 8 zum Hohlraum 3 des Milchbehälters 2 auf. Dieses verhindert, dass abgepumpte Milch aus dem Milchbehälter 2 wieder in das Verbindungselement 7 bzw. in die Brusthaube 4 zurückfließen kann. Wenn der Hohlraum 3 kein Entlüftungselement aufwiese, würde der Druck im Hohlraum 3 durch die eingebrachte Milch ansteigen und das weitere Abpumpen behindern. Daher weist die Brustpumpe 1 als Entlüftungselement einen Entlüftungskanal 9 auf, dessen erstes Ende 10 zum Hohlraum 3 des Milchbehälters 2 offen ist und dessen zweites Ende 11 offen zur Umgebung ist. Zumindest ein Abschnitt des Entlüftungskanals verläuft entlang des Verbindungselements 7. Dadurch, dass ein Entlüftungskanal 9 vorgesehen ist, dessen beiden Enden 10, 11 voneinander beabstandet sind, wird die Milchmenge verringert, die beispielsweise bei einer Schräghaltung der Brustpumpe 1 (z.B. beim Vornüberbeugen, Zurücklehen und/oder seitlichen Beugen der abpumpenden Person) durch den Entlüftungskanal 9 austritt. Das zweite Ende 11 ist dabei weiter vom Milchbehälter 2 entfernt als das erste Ende 10. Der Entlüftungskanal 9 verläuft dabei weg vom Hohlraum 3.

Im Konkreten ist das erste Ende 10 gebildet durch eine Öffnung bzw. einen Durchbruch 15 in einem Wandabschnitt des Verbindungselements 7 (siehe insbesondere Fig. 2), wobei die Öffnung 15 auf der einen Seite zum Hohlraum 3 und auf der anderen Seite zum Nippeltunnel 6 mündet. Daran anschließend verläuft der Entlüftungskanal 9 entlang eines Abschnitts, an dem das Verbindungselement 7 den Nippeltunnel 6 aufnimmt, also insbesondere entlang des Verbindungsabschnitts 17.

Insbesondere wird der Nippeltunnel an die Öffnung 15 anschließend gebildet durch eine erste Nut 16 im Nippeltunnel 6 (vgl. Fig. 3 und 4), die in Umfangsrichtung 20 des Nippeltunnels 6 verläuft. In dieser Ausführungsform verläuft die erste Nut 16 vollumfänglich. An die erste Nut 16 anschließend, insbesondere auf der der Öffnung 15 gegenüberliegenden Seite des Verbindungsabschnitts 17 bzw. 18, ist eine zweite Nut 19 im Nippeltunnel 6 vorgesehen, die in axialer Richtung 21 des Verbindungsabschnitts 17 bzw. 18 verläuft. Die zweite Nut 19 verläuft insbesondere von der ersten Nut 16 in Richtung der Brusthaube 4 bzw. in Richtung der Brust einer abpumpenden Person. Durch den in Umfangsrichtung 20 verlaufenden Abschnitt des Entlüftungskanals 9 erfolgt somit eine Verlagerung nach oben und durch den in axialer Richtung 21 verlaufenden Abschnitt erfolgt eine Verlagerung in Richtung der benutzenden Person, wodurch bei einem nach vorne beugen der benutzenden Person weniger oder gar keine Milch austritt.

Der Entlüftungskanal 9, insbesondere die zweite Nut 19, verläuft zu einer umfänglichen Kante 22 des Verbindungsabschnitts 17 (vgl. insb. Fig. 2), die mit dem Nippeltunnel 6 in Kontakt steht. Der Nippeltunnel 6 weist eine dritte Nut 23 auf, die in radialer Richtung 24 verläuft und über die der Entlüftungskanal 9 zur Umgebung mündet. Somit wird das zweite offene Ende 11 des Entlüftungskanals durch die dritte Nut 23 gebildet. In Summe liegt somit das zweite Ende 11 des Entlüftungskanals 9 in der Betriebsstellung beim Abpumpen oberhalb des ersten Endes 10 des Entlüftungskanals 9.

In einer alternativen, nicht gezeigten Ausführungsform kann (analog zur zweiten Nut 19) eine in axialer Richtung 21 verlaufende Nut an die Bohrung 15 anschließen bzw. mit dieser direkt in Verbindung stehen (diese Nut wäre somit an der Unterseite des Verbindungsabschnitts 17 vorgesehen), die in eine (analog zur ersten Nut 16) in Umfangsrichtung 20 verlaufende Nut mündet, die insbesondere entlang 180° in Umfangsrichtung 20 zur Oberseite des Verbindungsabschnitts 17 verläuft, die wiederum in eine in axiale Richtung 21 verlaufende Nut mündet, die zu der radial verlaufenden Nut 23 führt.

In einer weiteren alternativen, nicht gezeigten Ausführungsform kann statt der Nut 19 bzw. der Nut 23 auch eine axial verlaufende Nut im Verbindungselement 7 vorgesehen sein, die die in Umfangsrichtung 20 verlaufende Nut 16 über die umfängliche Kante 22 mit der Umgebung verbindet.

Der Nippeltunnel 6 weist auf seiner äußeren Mantelfläche (die im Wesentlichen mit dem Verbindungsabschnitt 17 des Verbindungselements 7 in Kontakt steht) zwei Dichtrippen 25 auf, von denen eine von der zweiten Nut 19 durchbrochen wird. Außerdem weist das Verbindungselement 7 einen Dichtring 26 zur Abdichtung gegenüber dem Milchbehälter 2 auf.

Die Anmelderin hat in Experimenten den Milchverlust bei einer erfindungsgemäßen Brustpumpe in der Ausführungsform der Fig. 1 gegenüber einer Brustpumpe, bei der zur Entlüftung eine Bohrung im Verbindungselement vorgesehen ist, die den Hohlraum direkt zur Umgebung verbindet, ermittelt. Für die Messungen wurde die Brustpumpe um 90° nach vorne gekippt (sodass die Brusthaube nach oben zeigt). Im durchgeführten Versuchsaufbau wurde der Einfluss unterschiedlicher Volumenströme der einströmenden Muttermilch (durch verschiedene Mütter, also unterschiedliche Milchmengen) nachgestellt. Dabei wurden drei verschiedene Querschnitte verwendet, die die Menge der von der Pumpe in den Milchbehälter geförderten Milch beeinflussen. Es wurde festgestellt, dass diese Menge einen Einfluss auf die zu erwartende Leckage hat. Außerdem wurde festgestellt, dass die erfindungsgemäße Ausgestaltung deutlich reduzierte Milchverluste aufweist.

| Querschnitt (beeinflusst geförderte Milch) | Brustpumpe mit Bohrung nach außen in Verbindungselement | erfindungsgemäße Brustpumpe |
|---|---|---|
| MAM Sauger 0 | 75-90 g | 9, 2 g |
| MAM Sauger 1 | 80 g | 12-14 g |
| Spritznadel 0,5 (Durchfluss=3ml/min) | 27,6 g | 6, 2 g |

Dabei erlaubt die Spritznadel die geringsten geförderten Milchmengen und MAM Sauger 1 die höchsten geförderten Milchmengen.

## Patentansprüche

1. Brustpumpe (1), aufweisend
- einen Milchbehälter (2) mit einem Hohlraum (3) zur Aufnahme von abgepumpter Milch;
- eine Brusthaube (4), die einen Brusttrichter (5) und einen Nippeltunnel (6) aufweist;
- ein Verbindungselement (7), das eine Öffnung (8) zum Hohlraum (3) des Milchbehälters (2) mit dem Nippeltunnel (6) verbindet;
**gekennzeichnet durch**
- einen Entlüftungskanal (9), dessen erstes Ende (10) zum Hohlraum (3) des Milchbehälters (2) offen ist und dessen zweites Ende (11) offen zur Umgebung ist, wobei zumindest ein Abschnitt des Entlüftungskanals (9) entlang des Verbindungselements (7) verläuft.

2. Brustpumpe (1) nach Anspruch 1, wobei das zweite Ende (11) des Entlüftungskanals (9) weiter vom Milchbehälter (2) entfernt ist als das erste Ende (10).

3. Brustpumpe (1) nach einem der vorherigen Ansprüche, wobei zumindest ein Abschnitt des Entlüftungskanals (9) in eine Richtung weg vom Hohlraum (3) verläuft.

4. Brustpumpe (1) nach einem der vorherigen Ansprüche, wobei der Entlüftungskanal (9) zumindest abschnittsweise entlang eines Abschnitts verläuft, an dem das Verbindungselement (7) den Nippeltunnel (6) aufnimmt.

5. Brustpumpe (1) nach einem der vorherigen Ansprüche, wobei das erste Ende (10) des Entlüftungskanals (9) durch eine Öffnung (15) in einem Wandabschnitt des Verbindungselements (7) gebildet ist, wobei die Öffnung (15) auf der einen Seite zum Hohlraum (3) und auf der anderen Seite zum Nippeltunnel (6) mündet.

6. Brustpumpe (1) nach einem der vorherigen Ansprüche, wobei das Verbindungselement (7) einen Verbindungsabschnitt (17) zur Verbindung mit dem Nippeltunnel (6) aufweist, wobei der Verbindungsabschnitt (17) allgemein zylinderförmig oder konisch ausgebildet ist, wobei der Entlüftungskanal (9) zumindest abschnittsweise in Umfangsrichtung (20) des Verbindungsabschnitts (17) des Verbindungselements (7) verläuft.

7. Brustpumpe (1) nach den Ansprüchen 5 und 6, wobei der Entlüftungskanal (9) zumindest abschnittsweise gebildet ist durch eine Nut (16) im Nippeltunnel (6), die in Umfangsrichtung (20) des Nippeltunnels (6) verläuft, wobei vorzugsweise die andere Seite der Öffnung (15) zur Nut (16) mündet.

8. Brustpumpe (1) nach Anspruch 5 und vorzugsweise einem der Ansprüche 6 und 7, wobei das Verbindungselement (7) einen Verbindungsabschnitt (17) zur Verbindung mit dem Nippeltunnel (6) aufweist, wobei der Verbindungsabschnitt (17) allgemein zylinderförmig oder konisch ausgebildet ist, wobei der Entlüftungskanal (9) zumindest abschnittsweise in axialer Richtung (21) des Verbindungsabschnitts (17) verläuft.

9. Brustpumpe (1) nach Anspruch 8, wobei der Entlüftungskanal (9) zumindest abschnittsweise gebildet ist durch eine Nut (19) im Nippeltunnel (6), die in axialer Richtung (21) des Verbindungsabschnitts (17) des Verbindungselements (7) verläuft.

10. Brustpumpe (1) nach Anspruch 8 oder 9, wobei der Entlüftungskanal (9) zu einer umfänglichen Kante (22) des Verbindungsabschnitts (17) des Verbindungselements (7) verläuft, die mit dem Nippeltunnel (6) in Kontakt steht, wobei die Kante (22) des Verbindungsabschnitts (17) und/oder der Nippeltunnel (6) eine radial verlaufende Nut (23) aufweist, über die der Entlüftungskanal (9) zur Umgebung mündet.

11. Brustpumpe (1) nach einem der vorhergehenden Ansprüche, wobei das zweite Ende (11) des Entlüftungskanals (9) in einer Betriebsstellung der Brustpumpe (1) zum Abpumpen von Milch oberhalb des ersten Endes (10) des Entlüftungskanals (9) liegt.

12. Brustpumpe (1) nach einem der vorhergehenden Ansprüche, wobei ein Rückschlagventil (14) an der Öffnung (8) zum Hohlraum (3) des Milchbehälters (2) vorgesehen ist.

13. Brustpumpe (1) nach einem der vorhergehenden Ansprüche, wobei das Verbindungselement (7) eine Membran (13) aufweist, über die ein Pumpelement einen Unterdruck im Verbindungselement (7) erzeugen kann.

14. Brustpumpe (1) nach Anspruch 13, aufweisend ein elektrisches Pumpenelement, das über die Membran (13) einen Unterdruck im Verbindungselement (7) erzeugen kann.
